(19) Europäisches Patentamt
European Patent Office
Office européen des brevets

(11) EP 3 011 951 B1

(12) EUROPEAN PATENT SPECIFICATION

(45) Date of publication and mention of the grant of the patent:
**07.08.2019 Bulletin 2019/32**

(21) Application number: **14812987.7**

(22) Date of filing: **10.06.2014**

(51) Int Cl.:
***A61K 8/49*** *(2006.01)* ***A61Q 5/06*** *(2006.01)*
***C09B 23/00*** *(2006.01)* ***C07D 209/14*** *(2006.01)*
***C09B 26/04*** *(2006.01)*

(86) International application number:
**PCT/JP2014/065379**

(87) International publication number:
**WO 2014/203771 (24.12.2014 Gazette 2014/52)**

(54) **YELLOW COLORANT FOR HAIR DYEING, COMPOSITION FOR HAIR DYEING, AND HAIR DYEING METHOD**

GELBER FARBSTOFF ZUR HAARFÄRBUNG, ZUSAMMENSETZUNG ZUR HAARFÄRBUNG SOWIE VERFAHREN ZUR HAARFÄRBUNG

COLORANT JAUNE POUR LA TEINTURE DES CHEVEUX, COMPOSITION POUR LA TEINTURE DES CHEVEUX, ET PROCÉDÉ DE TEINTURE DES CHEVEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.06.2013 JP 2013126630**

(43) Date of publication of application:
**27.04.2016 Bulletin 2016/17**

(73) Proprietor: **Hodogaya Chemical Co., Ltd.**
**Chuo-ku, Tokyo 104-0028 (JP)**

(72) Inventors:
- **AKIBA Mitsuhiro**
  **Yokohama-shi**
  **Kanagawa 230-0053 (JP)**
- **SUZUKI Kenichiro**
  **Yokohama-shi**
  **Kanagawa 230-0053 (JP)**
- **GONDA Michihiro**
  **Tokyo 104-0028 (JP)**
- **ISOBE Masatoshi**
  **Yokohama-shi**
  **Kanagawa 230-0053 (JP)**

(74) Representative: **Müller-Boré & Partner Patentanwälte PartG mbB**
**Friedenheimer Brücke 21**
**80639 München (DE)**

(56) References cited:
**EP-A2- 0 312 343** **EP-A2- 1 142 559**
**DE-A1- 3 829 870** **JP-A- H01 132 512**
**JP-A- 2001 288 057** **US-A- 4 760 132**
**US-A1- 2007 006 398**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to a composition for hair dyeing and a hair dyeing method. More precisely, the present invention relates to a composition for hair dyeing, which contains a cationic dye suitable for dyeing keratin fibers contained in human hair and domestic animal hair, and to a hair dyeing method using the composition for hair dyeing.

In general, hair dyeing can be roughly categorized into a chemical reaction type (oxidative hair colorants) in which an oxidative dye such as paraphenylenediamine is mixed with aqueous hydrogen peroxide in using whereupon melamine dye in hair is decolored by the hydrogen peroxide and at the same time the oxidative dye oxidatively colors for hair dyeing; and a physical adsorption type (hair colorants) in which a direct dye such as an acidic dye or a basic dye physically adsorbs to keratin fibers having positive or negative charges for hair dyeing.

A major part of hair dyeing is a dyeing method using an oxidative dye, in which a colorless precursor substance is applied to hair and the precursor substance is oxidatively polymerized to form a massive coloring substance. The method can secure stable long-lasting hair dyeing, in which, however, some diamine-type oxidative dyes may infrequently cause cutaneous allergic response (irritation) depending on individual's physical constitutions. In addition, as other components, the dyes may contain an alkali agent such as ammonia, and are therefore defective in that the dye may damage hair.

On the other hand, as typical products capable of temporarily coloring hair, there are mentioned hair manicures, color treatments and color rinses. The main dyes of these types are acidic dyes usable in cosmetics, which are advantageous in that, since the allergic response thereof is weak as compared with the previous oxidative dyes, even a person who would be irritated by oxidative hair colorants could use the dyes. In addition, the dyes do not use an alkali agent, they may poorly damage hair. However, as compared with oxidative dyes of chemical reaction type, the acidic dyes are defective in that they may often discolor in shampooing.

As the colorants for use in a hair manicure or the like for temporarily coloring hair, also usable are basic dyes and the like in addition to the acidic dyes, but the hair-coloring fastness of these dyes are moderate. Of those basic dyes, it is said that cationic dyes has a poor light-fastness and shampooing-fastness in hair dyeing.

Toning may be attained, for example, by combining three primary colors (yellow, red, blue) of dyes in any desired manner. In the case of reactive oxidative hair colorants, the blending ratio thereof with an oxidizing agent may be changed with respect to respective color number, but in many cases, the color in hair dyeing may differ from the color number. On the other hand, in the case of hair colorants such as a hair manicure, colored dyes are mixed and used, and the case is therefore advantageous in that the intended color number is easy to attain.

As the hair colorants for use in a hair manicure and the like, brown colorants are much used; however, of basic direct dyes to be used, there are few yellowish or yellow-orangey dyes that could be excellent in dyeing performance, color tone and fastness. In addition, of those, there are many mutagenicity-positive dyes, and there are a limited number of colorants for hair dyeing having high safety. Further, desired are inexpensive compositions for hair dyeing with good dyeing performance and good level dyeing capability.

As a method for rapidly dyeing keratin fibers of human hair or domestic animal hair by using the above-mentioned basic direct dyes, there are known a dyeing method using a triarylmethane dye, a cationic azo dye, a methine dye, an azomethine dye or the like in a solvent suitable for dyeing (e.g., see PTL 1), and a method of dyeing keratin-containing fibers, especially human hair with a cationic dye (e.g., see PTL 2).

CITATION LIST

PATENT LITERATURE

PTL 1: JP-A 2004-285048
PTL 2: JP-T H08-507545
PTL 3: JP-A S62-156165


EP 0 312 343 A2 discloses a hair treatment product.

US 2007/006398 A1 discloses a composition for dyeing keratin fibers, comprising at least one diamino-N,N-dihydropyrazolone derivative, at least one coupler, and at least one heterocyclic direct dye.

However, though cationic dyes have high dyeing performance and are therefore suitable for dyeing human hair and domestic animal hair, those sufficiently satisfactory in point of light-fastness and shampooing-fastness of dyed hair could not be obtained as yet at present.

An object of the present invention is to provide a colorant for hair dyeing, which is excellent in dyeing performance, has good solubility in water and is excellent in light-fastness and shampooing-fastness, and to provide a composition

for hair dyeing, which contains the same.

The present invention has been attained as a result of assiduous investigations for solving the above-mentioned problems. The subject matter of the present invention is characterized in the claims.

Specifically, the present invention provides a composition for hair dyeing comprising a colorant for hair dyeing, containing a compound represented by the following general formula (I):

[Chem. 1]

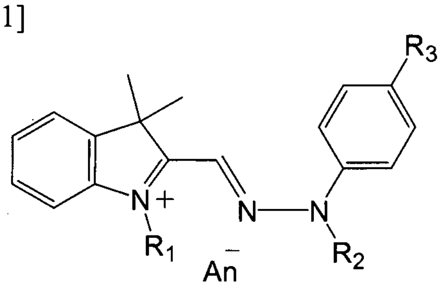

Formula (I)

(In the formula, $R_1$, $R_2$ and $R_3$ each independently represent a methyl group, and $An^-$ represents a chloride ion.)

The colorant for hair dyeing containing the compound represented by the general formula (I) belongs to an yellow cationic dye, but the present inventors have found that this compound is excellent in dyeing performance for keratin fibers contained in human hair, domestic animal hair and the like, has good solubility in water and is excellent in light-fastness and shampooing-fastness. In addition, when it is used along with a penetrant and a solvent combined therewith, the dyeing performance thereof is further improved.

The colorant for hair dyeing containing the compound represented by the general formula (I) has another advantage that it enables direct dyeing in an aqueous solution of the colorant at a practical temperature of from 15°C to 45°C even without using a penetrant and a solvent. Further, by purifying the colorant, a mutagenicity-negative and safe compound can be provided easily.

The compound represented by the general formula (I) can also be expressed as a resonant structure shown by the following general formula (I') (hereinafter the description of the resonant structure of the compound having the same skeleton as that of the general formula (I) is omitted).

[Chem. 2]

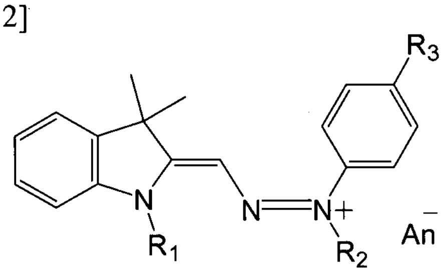

Formula (I')

(In the formula, $R_1$, $R_2$, $R_3$ and $An^-$ each have the same meanings as those described for the general formula (I).)

The general formula (I) represents the compound where $R_1$, $R_2$ and $R_3$ are methyl groups and $An^-$ is a chloride ion, which is a dye known as C. I. Basic Yellow 29 (CAS. Number 39279-59-9) represented by the following structural formula (II) (e.g., PTL 3). The dye is known to be able to dye acrylic fibers that are synthetic fibers by bonding to a sulfonic acid group. The present inventors have found that yellow colorants for hair dyeing containing the compounds represented by the general formula (I) have a high hair-dyeing density and are excellent in point of the light-fastness of the dyed substance, particularly excellent in point of the shampooing-fastness thereof, as compared with basic yellow dyes such as Basic Yellow 57 (compound represented by the following formula (III)), Basic Yellow 87 (compound represented by the following formula (IV)) or the like, which are already used for hair dyeing, and that they are therefore useful as a main ingredient for brown to black colorants for hair dyeing.

[Chem. 3]

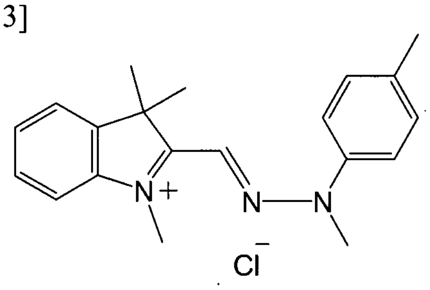

Formula (II)

[Chem. 4]

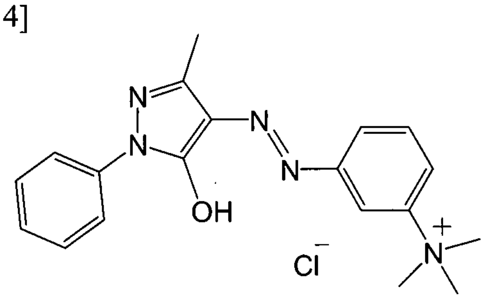

Formula (III)

[Chem. 5]

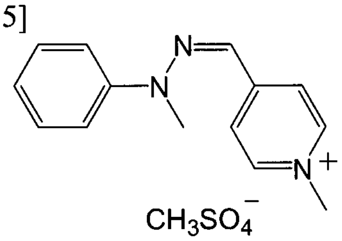

Formula (IV)

The present invention provides a composition for hair dyeing, which contains a colorant for hair dyeing, containing a compound represented by the above-mentioned general formula (I); at least one auxiliary agent selected from the group consisting of a moisturizer, a swelling agent, a penetrant, a solvent, a pH regulator, a fragrance, and a thickener; a surfactant, which is a cationic surfactant or a nonionic surfactant; and water.

The composition for hair dyeing can be used as a so-called hair colorant and can exhibit excellent dyeing performance, light-fastness and shampooing-fastness.

The present invention also provides a hair-dyeing method using the above-mentioned composition for hair dyeing.

According to the present invention, there is provided a composition for hair dyeing for wide-range colors of yellow, brown to black, by taking advantages of high level dyeing performance and good water-solubility characteristics of the cationic dyes including the compounds represented by the general formula (I). In addition, the composition for hair dyeing according to the present invention is especially excellent in shampooing-fastness and further has good light-fastness, as compared with cationic dyes of already-existing colorants for hair dyeing.

Moreover, the composition for hair dyeing according to the present invention is usable in a pH range of from 5 to 7. The colorants for hair dyeing containing the compound represented by the general formula (I) are excellent in dyeing performance and fastness and can uniformly dye hair, as yellow colorants by themselves, but when combined with any of red colorants for hair dyeing or blue colorants for hair dyeing, they can realize color-toning in yellow, brown and black. Accordingly, the colorant for hair dyeing containing a compound represented by the general formula (I) of the present invention can provide an inexpensive composition for hair dyeing that has good level dyeing capability and realizes wide-range color tone.

The details of embodiments of the present invention are described below. However, the present invention is not limited to the following embodiments.

The colorant for hair dyeing of the present invention contains a compound represented by the above-mentioned general formula (I), and is a yellow colorant categorized as a group of methine dyes. The compounds represented by the general formula (I) can be synthesized according to a known method, and for example, can be synthesized according to the following method.

An aniline derivative represented by the following general formula (i) is diazotized under an acidic condition in

water, then reacted with an indolenine derivative represented by the following formula (ii), subsequently quaternized with an alkylsulfate or the like, and salted out with salt or the like to give a compound represented by the general formula (I).

[Chem. 6]

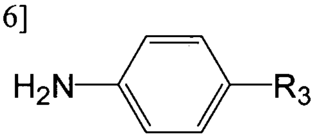

Formula (i)

[Chem. 7]

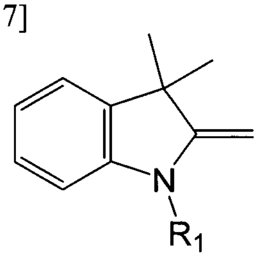

Formula (ii)

In the general formula (I), $R_1$, $R_2$ and $R_3$ each independently represent a methyl group.

In the general formula (I), $R_1$ is a methyl group. $R_2$ is a methyl group. $R_3$ is a methyl group.

In the general formula (I), as the combination of $R_1$, $R_2$ and $R_3$, $R_1$, $R_2$ and $R_3$ are all methyl groups.

In the general formula (I), $An^-$ represents an inorganic anion.

The inorganic anion specifically is a chloride ion. The inorganic anion can be introduced by adding hydrochloric acid to the dye after methine dye production.

$An^-$ is a chloride ion.

An embodiment of the composition for hair dyeing according to the present invention is an embodiment of a type of so-called hair colorants, and contains the colorant for hair dyeing containing the compound represented by the general formula (I); at least one auxiliary agent selected from the group consisting of a moisturizer, a swelling agent, a penetrant, a solvent, a pH regulator, a fragrance, and a thickener; a surfactant, which is a cationic surfactant or a nonionic surfactant; and water.

The moisturizer includes glycerin, propylene glycol, sorbitols, 1,3-butylene glycol, polyethylene glycols, and the like. In the case of using a moisturizer, the content thereof is preferably from 0.1 to 20% by mass and more preferably from 0.5 to 10% by mass, based on the total amount of the composition for hair dyeing.

The swelling agent includes an aqueous alkali solution such as ammonia (ammonium hydroxide) or monoethanolamine (MEA). In the case of using a swelling agent, the content thereof is preferably from 0.1 to 20% by mass and more preferably from 0.5 to 10% by mass, based on the total amount of the composition for hair dyeing.

As the penetrant and the solvent, there are mentioned monoalcohols having an alkyl group with from 1 to 6 carbon atoms, such as ethanol, 1-propanol, 2-propanol, 1-butanol, 2-butanol, and butoxyethanol; polyalcohols having from 3 to 8 carbon atoms and ethers thereof, such as propanediol, butanediol, pentanediol, hexanediol, hexanetriol, heptanediol, heptanetriol, octanediol, octanetriol, isoprene glycol, propylene glycol, glycerin, and diethylene glycol monoethyl ether; N-alkylpyrrolidones liquid at room temperature, such as N-methyl-2-pyrrolidone, N-ethyl-2-pyrrolidone, N-propyl-2-pyrrolidone, N-butyl-2-pyrrolidone, and N-cyclohexyl-2-pyrrolidone; alkylene carbonates (lower alkylene carbonates) such as ethylene carbonate, and propylene carbonate; aromatic alcohols such as benzyloxyethoxyethanol, benzyl alcohol, benzyloxyethanol, cinnamyl alcohol, p-anisyl alcohol, p-methylbenzyl alcohol, phenoxyethanol, phenoxyisopropanol, 2-benzylethanol, and β-phenylethyl alcohol; and the like. Of those, preferred are aromatic alcohols or N-alkylpyrrolidones, and more preferred are benzyl alcohol, benzyloxyethoxyethanol and benzyloxyethanol. In the case of using a penetrant or a solvent, the content thereof is preferably from 2 to 40% by mass and more preferably from 5 to 20% by mass, based on the total amount of the composition for hair dyeing.

The pH regulator includes acids such as phosphoric acid, lactic acid/sodium lactate, and citric acid/sodium citrate; and bases such as aqueous ammonia, sodium hydroxide, potassium hydroxide, and sodium carbonate. In the case of using a pH regulator, the content thereof is preferably from 0.1 to 10% by mass and more preferably from 0.5 to 5% by mass, based on the total amount of the composition for hair dyeing.

As the surfactant, used here is a cationic surfactant or a nonionic surfactant. Specific examples thereof include silicone compounds such as polysiloxane, polyoxyethylene alkyl ethers, polyoxyethylene fatty acid esters, polyglycerin fatty acid esters, aliphatic amines and quaternary ammonium salts thereof (trimethylstearylammonium chloride, etc.), sugar alcohol ethers such as sorbitol alkyl ethers, polyoxyethylene sorbitan fatty acid esters, and the like, to which, however, the present invention is not limited. Of those, preferred are polyoxyethylene sorbitan fatty acid esters. Use of

polyoxyethylene sorbitan fatty acid esters further improves the skin contamination-reducing effect (skin contamination-preventing capability).

As the polyoxyethylene sorbitan fatty acid esters, preferred is at least one selected from the group consisting of polyoxyethylene sorbitan monolaurate, polyoxyethylene sorbitan monopalmitate, polyoxyethylene sorbitan monostearate, and polyoxyethylene sorbitan monooleate.

The content of the surfactant is preferably from 0.1 to 20% by mass and more preferably from 0.5 to 10% by mass, based on the total amount of the composition for hair dyeing from the viewpoint of reducing skin contamination.

The fragrance includes vanillin, cinnamic alcohol, heliotropin, coumarin, 2-methyl-3-(3,4-methylenedioxy-phenyl)-propanal, 4-(4-hydroxyphenyl)-2-butanone, benzaldehyde, anise alcohol, 3,4-dimethoxybenzaldehyde, heliotropyl acetate, phenylacetaldehyde dimethyl acetal, phenoxyethyl alcohol, phenylacetaldehyde glyceryl acetal, furaneol, sugar lactone, maltol, ethylmaltol, ethyl diglycol, benzyl acetate, linalool, camphor, terpineol, citronellol, geraniol, 2,6-nonadienal, methyloctyl carbonate, 3,7-dimethyl-2,6-octadienal, nonanal, and the like. In the case of using a fragrance, the content thereof is preferably from 0.00001 to 2% by mass based on the total amount of the composition for hair dyeing.

The thickener includes guar gum and derivatives thereof, hydroxyethyl cellulose, xanthane gum, collagen, gelatin, carboxymethyl cellulose sodium salt, Carbopol (registered trademark), sodium alginate, gum arabic, cellulose derivatives, and poly(ethyleneoxide)-derived thickeners. These thickeners have an effect of increasing the viscosity of the composition for hair dyeing to make the composition into a form of readily-handleable gel. In the case of using a thickener, the content thereof is preferably from 0.1 to 20% by mass and more preferably from 0.5 to 10% by mass, based on the total amount of the composition for hair dyeing.

Water to be in the composition for hair dyeing of the present invention is not specifically defined, for which usable is any of ion-exchanged water, purified water, clarified water, and the like.

The colorant for hair dyeing containing a compound represented by the general formula (I) of the present invention is excellent in dyeing performance and fastness as a yellow colorant for hair dyeing by itself, and can dye hair uniformly. In addition, when the colorant for hair dyeing containing a compound represented by the general formula (I) is combined with a blue colorant for hair dyeing and a red colorant for hair dyeing, color toning can be realized in yellow, brown and black.

The blue colorant for hair dyeing to be combined includes basic dyes such as Basic Blue 75 and 99; disperse dyes such as Disperse Blue 3, 7 and 377; HC dyes such as HC Blue 2, 7, 11, 12, 14, 15, 16, and 17; and the like. Preferred is Basic Blue 75.

The red colorant for hair dyeing to be combined includes basic dyes such as Basic Red 51 and 76; disperse dyes such as Disperse Red 11, 15 and 17; and HC dyes such as HC Red 1, 3, 7, 10, 11, 13, and 16. Preferred is Basic Red 51.

In place of such a blue colorant for hair dyeing or a red colorant for hair dyeing, also usable is a violet colorant for hair dyeing. The violet colorant for hair dyeing includes basic dyes such as Basic Violet 1, 2 and 14; disperse dyes such as Disperse Violet 1, 4, 8, 11, 13, and 15; and HC dyes such as HC Violet 1 and 2.

In the embodiment of the composition for hair dyeing of the present invention, it is preferred that the amount of the colorant for hair dyeing containing a compound represented by the general formula (I) is set to from 0.001 to 5% by mass based on the total amount of the composition for hair dyeing and the balance is made up by of at least one auxiliary agent selected from the group consisting of a moisturizer, a wetting agent, a penetrant, a solvent, a pH regulator, a fragrance, and a thickener, a surfactant, which is a cationic surfactant or a nonionic surfactant, and water. When the content of the colorant for hair dyeing is less than 0.001% by mass, it could hardly express the effect of maintaining color tone and level dyeing capability, but even when the adding amount exceeds 5% by mass, the effect thereof to improve the dyeing performance may be small. The content of the colorant for hair dyeing is preferably from 0.01 to 5% by mass and more preferably from 0.05 to 2% by mass, based on the total amount of the composition for hair dyeing.

The pH value of the composition for hair dyeing of the present invention is from 5 to 7. The pH value of the composition for hair dyeing can be controlled according to any known method, and is preferably controlled by using a pH regulator such as citric acid monohydrate, and trisodium citrate dihydrate. Specifically, in a case where a composition for hair dyeing having a pH of 6 is prepared, citric acid monohydrate or trisodium citrate dihydrate is previously dissolved in water to prepare an aqueous solution having a pH of 6, and then the colorant for hair dyeing including the compound represented by the general formula (I) and optionally other additives (auxiliary agent, etc.) are added thereto to give a composition for hair dyeing having a pH of 6.

Any known components for cosmetics may be added to the composition for hair dyeing of the present invention within a range not detracting from the advantageous effects of the present invention. The components for cosmetics that may be added include higher alcohols, vaseline, polyalcohols, esters, preservatives, microbicides, silicone derivatives, water-soluble polymers, and the like.

The hair dyeing method of the present invention uses the above-mentioned composition for hair dyeing of the present invention. Specifically, for example, a subject to be hair-dyed, such as human hair, domestic animal hair or the like is brought into contact with the composition for hair dyeing of the present invention for dyeing the hair. The hair-dyeing temperature is preferably from 5 to 60°C, and considering that it is carried out around scalp, the temperature is preferably from 15 to 45°C. The hair-dyeing time is preferably from 5 to 60 minutes, and more preferably from 10 to 30 minutes.

After dyed, the hair is usually post-treated by rinsing with water, drying, or the like. Rinsing with water may be carried out until the color of the hair colorant could not elute any more, and may be carried out, for example, by rinsing with running water of from 5 to 15 L/min at from 5 to 40°C for 0.5 to 2 minutes. Drying after the rinsing with water may be spontaneous drying (generally at 5 to 40°C for 10 minutes to 10 hours), or, if desired, a hot-air drier may be used (generally at 40 to 60°C, for 10 minutes to 10 hours).

After thus rinsed with water, soaping may be carried out. For the soaping, for example, a suitable amount of a soaping liquid (mixture of shampoo and lukewarm water) is used, and in general, the hair is washed therewith at a temperature of from 15 to 50°C for 1 to 10 minutes and then further rinsed until the soaping liquid could be completely removed.

EXAMPLES

The present invention is described in more detail with reference to Examples hereinunder, but the present invention is not whatsoever restricted by the following Examples. Examples 1 to 7 represent Reference Examples.

In the following Examples and Comparative Examples, the colorant names are abbreviated as follows.

C.I. Basic Yellow 29: Yellow 29
C.I. Basic Yellow 57: Yellow 57
C.I. Basic Yellow 87: Yellow 87
C.I. Basic Brown 16: Brown 16
C.I. Basic Red 51: Red 51
C.I. Basic Blue 75: Blue 75

In the following Examples and Comparative Examples, the test samples obtained were evaluated by using a spectrophotometer (Color Technosystem, JS555). The dyeing density (K/Sd) was calculated according to the following process. The reflectance ($R_\lambda$) at an indicated wavelength ($\lambda$) of the test sample before dyeing (gray hair) and after dyeing (dyed hair) was measured with the spectrophotometer, and according to the following Kubelka-Munk equation, the optical density (K/S) was calculated. The value calculated by subtracting the optical density (K/S) of the gray hair from the optical density (K/S) of the dyed hair is referred to as the dyeing density (K/Sd).

Kubelka-Munk equation:

$$K/S = \Sigma(1 - R_\lambda)^2/2R_\lambda$$

$R_\lambda$: reflectance at wavelength ($\lambda$),
$\lambda$: 400 to 700 nm (at intervals of 10 nm).

Regarding the color, L*, a* and b* were measured by using the spectrophotometer and using a color coordinate system CIE L*a*b*. The L* indicates brightness and larger value means a lower coloration intensity. The a* and b* are chromaticity each indicating hue and saturation. The a* corresponds to the pair axis of red/green, and a positive value means red and a negative value means green. The b* corresponds to the pair axis of yellow/blue, and a positive value means yellow and a negative value means blue.

The color (L*, a*, b*) of the test sample before dyeing and after dyeing was measured, and from the value of the difference ΔL*, Δa* and Δb*, the color difference ΔE* was calculated according to the following equation.

$$\Delta E^* = \{(\Delta L^*)^2 + (\Delta a^*)^2 + (\Delta b^*)^2\}^{1/2}$$

Example 1

As shown in Table 1, 1 g of citric acid monohydrate and 11 g of trisodium citrate dihydrate as pH regulators were dissolved in ion-exchanged water to prepare 1 kg of an aqueous solution (water + pH regulator) having a pH of 6. In the resultant aqueous solution having a pH of 6 were added Yellow 29 as a dye and benzyloxyethoxyethanol as a penetrant in the ratio as shown in Table 2 to prepare a composition for hair dyeing having a pH of 6. A 10 g of the thus-prepared composition for hair dyeing was metered, and 1 g of human gray hair (100%, Beaulax, Item Code BM-W-A) was put thereinto and dyed at 45°C for 20 minutes. The resultant dyed hair was rinsed with water (running water at 15 to 25°C, for about 1 minute), then soaped under the soaping condition mentioned below, further rinsed with water (running water at 15 to 25°C, for about 1 minute), and then spontaneously dried at room temperature (about 20°C) to prepare a test sample. In the following Examples and Comparative Examples, the pH of the composition for hair dyeing was controlled in the manner as in Table 1. In the Table, the concentration (g/kg) indicates the concentration of the pH regulator in the aqueous solution.

(Soaping Condition)

Soaping liquid: aqueous 5% sodium laurylsulfate solution
Bath ratio: 1/10 (mass of soaping liquid relative to 1 g mass of dyed hair)
Processing temperature: 45°C
Processing time: 5 minutes

[Table 1]

| pH | pH Regulator | Concentration (g/kg) |
|---|---|---|
| 5 | Citric acid monohydrate | 3.5 |
| | Trisodium citrate dihydrate | 9 |
| 6 | Citric acid monohydrate | 1 |
| | Trisodium citrate dihydrate | 11 |
| 7 | Potassium phosphate ($KH_2PO_4$) | 3 |
| | Disodium phosphate ($Na_2HPO_4 \cdot 12H_2O$) | 11.5 |

[Table 2]

| Component | Compounding Ratio (% by mass) |
|---|---|
| Dye | 0.2 |
| Benzyloxyethoxyethanol | 8 |
| Water + pH Regulator | balance |
| Total | 100 |

Comparative Example 1

A test sample was prepared according to the same method as in Example 1 except that Yellow 57 was used in place of Yellow 29 as the dye. Here, Yellow 57 is a dye shown by the following formula (III), which is listed in Category II in "List of Colorants Incorporable in Hair-Dyeing Materials" by Japan Hair Color Industry Association (JHCIA), and is a compound commercially available as Arianor Straw Yellow.

[Chem. 8]

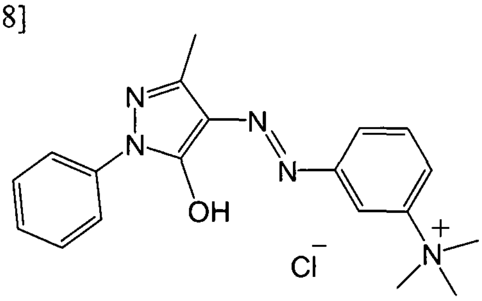

Formula (III)

Comparative Example 2

A test sample was prepared according to the same method as in Example 1 except that Yellow 87 was used in place of Yellow 29, as the dye. Here, Yellow 87 is a commercially available dye shown by the following formula (IV), which is listed in Category III in "List of Colorants Incorporable in Hair-Dyeing Materials" by Japan Hair Color Industry Association (JHCIA).

[Chem. 9]

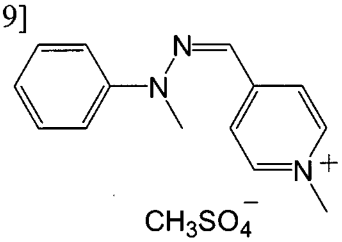

Formula (IV)

The test samples prepared in Example 1, Comparative Example 1 and Comparative Example 2 were analyzed with the spectrophotometer to measure the dyeing density (K/Sd) and the color values (L*, a*, b*) thereof. The results are shown in Table 3.

[Table 3]

| | K/Sd | L* | a* | b* |
|---|---|---|---|---|
| Example 1 (Yellow 29) | 96.5 | 68.98 | 18.83 | 72.42 |
| Comparative Example 1 (Yellow 57) | 28.5 | 74.73 | 1.68 | 47.03 |
| Comparative Example 2 (Yellow 87) | 65.5 | 74.04 | 1.64 | 68.74 |

Yellow 29 exhibited standard yellow as shown in Table 3, and the dyeing performance thereof was good. On the other hand, Yellow 57 was pale yellow, and Yellow 87 was slightly pale yellow.

Example 2 (Shampooing-Fastness Test)

The test sample with Yellow 29 prepared in Example 1 was tested according to the shampooing-fastness test mentioned below, and the dyeing density (K/Sd) and color difference (ΔE*) before and after the test were determined. The results are shown in Table 4. In the Table, the residual ratio (%) means the ratio of the dyeing density after the test to the dyeing density before the test.

(Shampooing-Fastness Test)

The test sample was subjected to a cycle operation of (1) shampooing followed by (2) treatment as mentioned below, repeatedly five times in all, and then spontaneously dried at room temperature (about 20°C).

(1) Shampooing Condition
Shampoo liquid: aqueous 5% sodium laurylsulfate solution
Bath ratio: 1/10 (mass of shampooing liquid relative to 1 g mass of dyed hair)
Processing temperature: 45°C
Processing time: 20 minutes
Post-treatment: rinsing with water
(2) Treatment Condition
Treatment liquid: aqueous 5% trimethylstearylammonium chloride solution
Bath ratio: 1/10 (mass of treatment liquid relative to 1 g mass of dyed hair)
Processing temperature: 45°C
Processing time: 20 minutes
Post-treatment: rinsing with water

Comparative Example 3

The test sample with Yellow 87 prepared in Comparative Example 2 was subjected to the same shampooing-fastness test as in Example 2, and the dyeing density (K/Sd) and color difference (ΔE*) before and after the test were determined. The results are shown in Table 4.

[Table 4]

| | Example 2 (Yellow 29) | | Comparative Example 3 (Yellow 87) | |
|---|---|---|---|---|
| | before test | after test | before test | after test |
| K/Sd | 96.5 | 79.2 | 65.5 | 21.8 |
| Residual Ratio % | 82 | | 33 | |
| L* | 68.98 | 71.12 | 74.04 | 75.22 |
| a* | 18.83 | 12.62 | 1.64 | -3.04 |
| b* | 72.42 | 71.99 | 69.74 | 48.46 |
| Color Difference (ΔE*) | 6.6 | | 21.8 | |

As shown in Table 4, as compared with the commercial product of Yellow 87, Yellow 29 remained in a high residual ratio and exhibited a small color difference. This confirms that it is a dye extremely excellent in shampooing-fastness.

Example 3, Example 4

Aqueous solutions having a pH of 5 and 7 were prepared in accordance with Table 1, and test samples were prepared according to the same method as in Example 1 (dye: Yellow 29) except that the aqueous solutions having a pH of 5 are 7 was used, respectively, in place of the aqueous solution having a pH of 6. The dyeing density (K/Sd) and the color values (L*, a*, b*) of the resultant test samples were determined. The results are shown in Table 5.

Example 5, Example 6

Test samples were prepared according to the same method as in Example 3 and Example 4 except that benzyloxyethoxyethanol (penetrant) in Example 3 and Example 4 was not used, respectively. The dyeing density (K/Sd) and the color values (L*, a*, b*) of the resultant test samples were determined. The results are shown in Table 5.

[Table 5]

| Example | pH | Penetrant | K/Sd | L* | a* | b* |
|---|---|---|---|---|---|---|
| Example 3 | 5 | presence | 80.1 | 71.42 | 14.09 | 72.89 |
| Example 4 | 7 | presence | 94.5 | 69.35 | 19.50 | 71.66 |
| Example 5 | 5 | - | 85.8 | 69.05 | 18.05 | 69.47 |
| Example 6 | 7 | - | 100.8 | 68.02 | 21.42 | 70.09 |

As shown in Table 5, Yellow 29 exhibited a high dyeing density even when the pH was 5 or the pH was 7, which confirms that the dye has excellent dyeing performance in a wide pH range. In addition, Yellow 29 exhibited a high dyeing density even in the case of using no penetrant, which also confirms the excellent dyeing performance of the dye.

Example 7 (Light-fastness Test)

A composition for hair dyeing having a pH of 6 was prepared according to the compounding formation as shown in Table 6 by using Yellow 29 as the dye. By using the thus-prepared composition for hair dyeing, a test sample was prepared according to the same method as in Example 1. The resultant test sample was exposed to white light at an

irradiation dose of 330 W/$m^2$ for 20 hours, by using a xenon long-life fade meter light-fastness tester (Suga Test Instrument, FAL-25AX-HC-BEC Model), and the dyeing density (K/Sd) and the color values (L*, a*, b*) before and after the test, and the color difference (ΔE*) were determined. The light-fastness test was carried out under the condition where the temperature of the test sample could be from 60 to 66°C. The results are shown in Table 7.

[Table 6]

| Component | Compounding Ratio (% by mass) |
|---|---|
| Dye | 0.2 |
| Benzyl Alcohol | 8 |
| Ethanol | 8 |
| Water + pH Regulator | balance |
| Total | 100 |

Comparative Example 4

A test sample was prepared according to the same method as in Example 7 except that Yellow 57 was used in place of Yellow 29, as the dye. The resultant test sample was subjected to the same light-fastness test as in Example 7, and the dyeing density (K/Sd) and the color values (L*, a*, b*) before and after the test, and the color difference were determined. The results are shown in Table 7.

[Table 7]

| | Example 7 (Yellow 29) | | Comparative Example 4 (Yellow 57) | |
|---|---|---|---|---|
| | before test | after test | before test | after test |
| K/Sd | 84.6 | 75.1 | 30.4 | 29.7 |
| Residual Ratio % | 89 | | 98 | |
| L* | 71.38 | 71.33 | 74.81 | 69.71 |
| a* | 15.36 | 13.79 | 2.08 | 0.98 |
| b* | 75.08 | 72.82 | 49.92 | 41.17 |
| Color Difference (ΔE*) | 2.8 | | 10.2 | |

As shown in Table 7, it can be found that Yellow 29 has a small color difference and is a dye excellent in light-fastness. On the other hand, Yellow 57 had a large color difference of 10.2, and it can be found that the light-fastness thereof is low.

Example 8 to Example 10

Compositions for hair dyeing having a pH of 6 were prepared according to the compounding formulation shown in Table 8 by using three color dyes of Yellow 29, Red 51 and Blue 75. By using the thus-prepared compositions for hair dyeing, test samples were prepared according to the same method as in Example 1. Each test sample was subjected to a measurement using the spectrophotometer, and the dyeing density (K/Sd) and the color values (L*, a*, b*) thereof were determined. The results are shown in Table 8.

[Table 8]

| Item | | Compounding Ratio (% by mass) | | |
|---|---|---|---|---|
| | | Example 8 | Example 9 | Example 10 |
| Formulation | Yellow 29 | 0.28 | 0.32 | 0.16 |
| | Red 51 | 0.08 | 0.10 | 0.19 |
| | Blue 75 | 0.008 | 0.06 | 0.15 |
| | Benzyloxyethoxyethanol | 8.0 | 8.0 | 8.0 |
| | Trimethylstearylammonium Chloride | 1.0 | 1.0 | 1.0 |
| | Hydroxyethyl Cellulose | 1.0 | 1.0 | 1.0 |
| | Water + pH Regulator | balance | balance | balance |
| | Total | 100 | 100 | 100 |
| Evaluation Results | K/Sd | 135.36 | 215.10 | 288.58 |
| | L* | 43.24 | 30.30 | 25.69 |
| | a* | 17.13 | 5.78 | 0.43 |
| | b* | 29.69 | 8.13 | 1.14 |
| | color tone | light brown | dark brown | natural black |

As shown in Table 8, it can be found that the incorporation of Red 51 and Blue 75 into Yellow 29 makes possible to dye into brown to black (light brown to natural black).

Example 11 to Example 13

Gel-type compositions for hair dyeing having a pH of 6 were prepared according to the compounding formulation shown in Table 9 by using three color dyes of Yellow 29, Red 51 and Blue 75. A 2 g of the thus-prepared composition for hair dyeing was metered, and uniformly applied to 1 g of human gray hair (100%, Beaulax, Item Code BM-W-A) and dyed it at 45°C for 20 minutes. The resultant dyed hair was rinsed with water (running water at 15 to 25°C, for about 1 minute), then soaped under the above-mentioned soaping condition, further rinsed with water (running water at 15 to 25°C, for about 1 minute), and then spontaneously dried at room temperature (about 20°C) to prepare a test sample. Each test sample was subjected to a measurement using the spectrophotometer, and the dyeing density (K/Sd) and the color values (L*, a*, b*) thereof were determined. The results are shown in Table 9.

[Table 9]

| Item | | Compounding Ratio (% by mass) | | |
|---|---|---|---|---|
| | | Example 11 | Example 12 | Example 13 |
| Formulation | Yellow 29 | 0.280 | 0.300 | 0.165 |
| | Red 51 | 0.030 | 0.110 | 0.150 |
| | Blue 75 | 0.010 | 0.090 | 0.185 |
| | Benzyloxyethoxyethanol | 8.0 | 8.0 | 8.0 |
| | Trimethylstearylammonium Chloride | 1.0 | 1.0 | 1.0 |
| | Guar Hydroxypropyltrimonium Chloride | 1.0 | 1.0 | 1.0 |
| | Hydroxyethyl Cellulose | 1.0 | 1.0 | 1.0 |
| | Water + pH Regulator | balance | balance | balance |
| | Total | 100 | 100 | 100 |

(continued)

| Item | | Compounding Ratio (% by mass) | | |
|---|---|---|---|---|
| | | Example 11 | Example 12 | Example 13 |
| Evaluation Results | K/Sd | 130.7 | 191.3 | 240.5 |
| | L* | 44.45 | 31.68 | 28.03 |
| | a* | 18.04 | 3.95 | 0.30 |
| | b* | 32.31 | 7.39 | 1.87 |
| | color tone | light brown | dark brown | natural black |

As shown in Table 9, it can be found that the incorporation of Red 51 and Blue 75 into Yellow 29 makes possible to dye into brown to black (light brown to natural black) even in a practicable gel-type composition.

The present application is based on Japanese Patent Application 2013-126630 filed on June 17, 2013.

The colorant for hair dyeing contained in the composition for hair dyeing of the present invention containing a compound represented by the general formula (I) can tone the color of keratin fibers such as human hair and the like in standard yellow to black, singly or as combined with any already-existing cationic dye. The colorant for hair dyeing has high dyeing performance and good light-fastness and is a colorant for hair dyeing especially excellent in shampooing-fastness, and therefore the colorant can provide an inexpensive composition for hair dyeing having good level dyeing capability and having a wide-range color tone.

## Claims

1. A composition for hair dyeing comprising:

   a colorant for hair dyeing, comprising a compound represented by the following general formula (I);
   at least one auxiliary agent selected from the group consisting of a moisturizer, a swelling agent, a penetrant, a solvent, a pH regulator, a fragrance, and a thickener;
   a surfactant, which is a cationic surfactant or a nonionic surfactant; and
   water,
   wherein the composition has a pH value of from 5 to 7:

   [Chem. 1]

   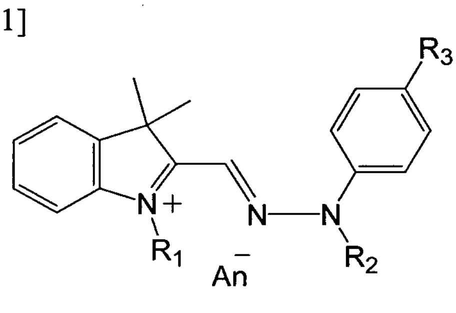

   Formula (I)

   in the formula, $R_1$, $R_2$ and $R_3$ each independently represent a methyl group, and $An^-$ represents a chloride ion.

2. A hair dyeing method using the composition for hair dyeing described in claim 1.

## Patentansprüche

1. Zusammensetzung zum Haarefärben, umfassend:

   ein Färbemittel zum Haarefärben, umfassend eine Verbindung, dargestellt durch die folgende allgemeine Formel (I);
   mindestens einen Hilfsstoff, ausgewählt aus der Gruppe, bestehend aus einem Befeuchtungsmittel, einem

Quellmittel, einem Eindringmittel, einem Lösungsmittel, einem pH-Wert-Regulierer, einem Duftstoff und einem Verdickungsmittel;
ein grenzflächenaktives Mittel, welches ein kationisches grenzflächenaktives Mittel oder ein nichtionisches grenzflächenaktives Mittel ist; und
Wasser,
wobei die Zusammensetzung einen pH-Wert von 5 bis 7 aufweist:

[Chem. 1]

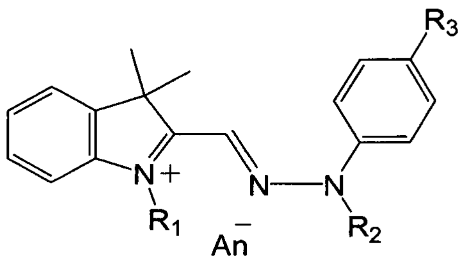

Formel (I)

in der Formel stellt jedes von $R_1$, $R_2$ und $R_3$ unabhängig eine Methylgruppe dar und stellt $An^-$ ein Chloridion dar.

**2.** Verfahren zum Haarefärben unter Verwendung der Zusammensetzung zum Haarefärben nach Anspruch 1.

## Revendications

**1.** Composition pour teinture de cheveux comprenant :

un colorant pour teinture de cheveux comprenant un composé représenté par la formule générale suivante (I) ;
au moins un agent auxiliaire sélectionné parmi le groupe constitué d'un hydratant, d'un agent gonflant, d'un agent pénétrant, d'un solvant, d'un régulateur de pH, d'un parfum et d'un épaississant ;
un tensioactif qui est un tensioactif cationique ou un tensioactif non ionique ; et
de l'eau,
dans laquelle la composition a une valeur de pH de 5 à 7 :

[chim. 1]

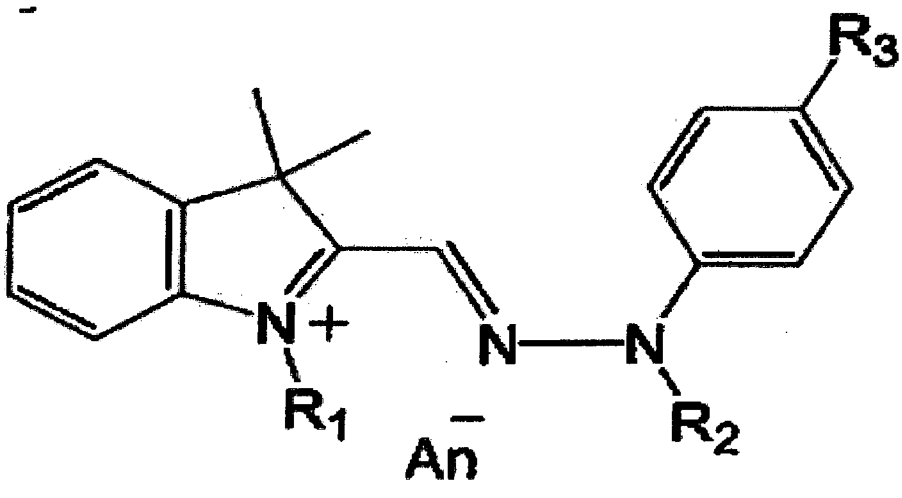

Formule (I)

dans la formule, $R_1$, $R_2$ et $R_3$ représentent chacun indépendamment un groupe méthyle et $An^-$ représente un ion chlorure.

**2.** Procédé de teinture de cheveux utilisant la composition pour teinture de cheveux décrite à la revendication 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004285048 A
- JP H08507545 T
- JP S62156165 A
- EP 0312343 A2
- US 2007006398 A1
- JP 2013126630 A